# EUROPÄISCHE PATENTSCHRIFT

(11) **EP 3 402 538 B1**
(45) Veröffentlichungstag und Bekanntmachung des Hinweises auf die Patenterteilung: **16.03.2022**
(21) Anmeldenummer: 16825992.7
(22) Anmeldetag: 16.12.2016
(51) Int. Cl.: A61L 2/04, F16L 101/40

(54) **VORRICHTUNG ZUM ABTÖTEN VON KEIMEN UND/ODER KRANKHEITSERREGERN**
DEVICE FOR KILLING GERMS AND/OR PATHOGENIC AGENTS
DISPOSITIF POUR TUER DES GERMES ET/OU DES AGENTS PATHOGÈNES

(30) Priorität: 14.01.2016 AT 92016
(43) Veröffentlichungstag der Anmeldung: 21.11.2018
(73) Patentinhaber: Arbeiter, Peter, 6932 Langen (AT)
(72) Erfinder: Arbeiter, Peter, 6932 Langen (AT)
(74) Vertreter: Torggler & Hofmann Patentanwälte - Rankweil
(86) Internationale Anmeldenummer: PCT/AT2016/000101
(87) Internationale Veröffentlichungsnummer: WO 2017/120628

(56) Entgegenhaltungen:
- EP-A1- 2 868 330
- DE-A1- 19 543 503
- DE-A1- 19 914 676
- DE-A1-102009 020 932
- DE-C1- 4 216 571

## Beschreibung

Vorrichtung zum Abtöten von Keimen und/oder Krankheitserregern in einem pumpfähigen Substrat, wobei die Vorrichtung zumindest einen Behälter mit einem, von einer Behälterwand des Behälters umgebenen, Behälterhohlraum zur Aufnahme des pumpfähigen Substrats aufweist, und im Behälterhohlraum zumindest ein innerhalb des Behälterhohlraums verlagerbarer Trennkörper der Vorrichtung angeordnet ist, wobei der Trennkörper den Behälterhohlraum in zwei voneinander getrennte Teilvolumina zur Aufnahme des pumpfähigen Substrats trennt.

Vorrichtungen dieser Art werden generell dann eingesetzt, wenn in pumpfähigen Substraten Keime und/oder Krankheitserreger, welcher Art auch immer, abgetötet werden müssen. Dies ist in der Lebensmittelindustrie an manchen Stellen genauso notwendig wie z.B. auch bei der Verarbeitung von pumpfähigen Substraten mit organischen Anteilen in Biogasanlagen und dergleichen. Bei vielen dieser Einsatzgebiete müssen ausreichende Verweilzeiten des zu behandelnden pumpfähigen Substrats im Behälter sichergestellt werden, damit die jeweils zu beseitigenden bzw. abzutötenden Keime und/oder Krankheitserreger auch sicher in der gewünschten Art und Weise beseitigt bzw. abgetötet wurden. Hierzu gibt es in vielen Bereichen gesetzliche Vorschriften, die überprüfbar eingehalten werden müssen.

Vorrichtungen der oben genannten Art sind beim Stand der Technik in verschiedensten Ausgestaltungsformen bekannt. Als Beispiele hierzu sind sogenannte Batch-Verfahren zu nennen. Dafür geeignete Vorrichtungen sind z.B. in der EP 2 868 330 A1 und der EP 2 253 335 B1 gezeigt. Die DE 42 00 588 A1 offenbart eine Vorrichtung zur Sterilisation von pumpfähigen Substraten, bei der der Behälter in Form einer Rohrwendel ausgeführt ist. Die WO 00/04934 A1 zeigt eine Vorrichtung, bei der der Behälter rohrartig ausgebildet ist und in diesem Rohr eine Förderschnecke angeordnet ist.

Die DE 42 16 571 C1 beschreibt eine Vorrichtung zur Sterilfiltration erwärmter Medien wie Wasser mit einem Molch, der sich in einem Kreislauf mit Zu- und Ablauf bewegen lässt.

Die DE 10 2009 020 932 A1 beschreibt eine Umkehrosmoseanlage, bei der in Druckrohren angeordnete Molche mit Hilfe von Linearantrieben verschoben werden. Die DE 195 43 503 A1 offenbart ein Verfahren und eine Vorrichtung zum Bestrahlen von bewegten Flüssigkeiten in einer Rohrschlange, wobei die Strahlung von zumindest einem, von der Flüssigkeit mitgeführten Strahlendosimeter gemessen wird.

Die DE 199 14 676 A1 beschreibt einen Verschlussdeckel zur Erzeugung eines Vakuums.

Aufgabe der Erfindung ist es, eine Vorrichtung der oben genannten Art vorzuschlagen, bei der die zur Abtötung der Keime und/oder Krankheitserreger benötigten bzw. gegebenenfalls auch gesetzlich vorgeschriebenen Verweilzeiten des pumpfähigen Substrats im Behälterhohlraum sichergestellt werden können.

Diese Aufgabe löst die Erfindung durch eine Vorrichtung gemäß Patentanspruch 1. Ein Grundgedanke der Erfindung ist es, dass man durch den im Behälterhohlraum verlagerbaren bzw. verschiebbaren Trennkörper sicherstellt, dass sich pumpfähiges Substrat aus einem Teilvolumen, welches sich auf einer Seite des Trennkörpers im Behälterhohlraum befindet, nicht über den Trennkörper hinaus in ein anderes Teilvolumen im Behälterhohlraum ausbreiten kann. Hierdurch wird eine überwachbare und nachvollziehbare Grenze für das jeweilige Teilvolumen des pumpfähigen Substrats im Behälterhohlraum geschaffen, anhand der nachgewiesen und überprüft werden kann, wie lange sich das jeweilige Teilvolumen an pumpfähigem Substrat zum Abtöten der Keime und/oder Krankheitserreger im Behälterhohlraum befunden hat. Damit kann verhindert werden, dass bereits ausreichend lang behandeltes pumpfähiges Substrat noch einmal durch den Kontakt mit noch nicht ausreichend lang behandeltem Substrat kontaminiert wird. In verschiedenen Ausführungsformen der Erfindung können auch mehrere Trennkörper in einem Behälterhohlraum, vorzugsweise hintereinander und voneinander distanziert, vorhanden sein. Auch in diesem Fall trennt jeder Trennkörper den Behälterhohlraum in zwei Teilvolumina, wobei dann eben diese Teilvolumina durch wieder andere der Trennkörper in weitere Teilvolumina getrennt werden. Bevorzugte Varianten der Erfindung sehen vor, dass der Trennkörper eine Barriere zur Verhinderung der Vermischung von pumpfähigem Substrat aus einem der Teilvolumina mit pumpfähigem Substrat aus dem anderen der Teilvolumina ist. Besonders bevorzugt ist in diesem Zusammenhang vorgesehen, dass der Trennkörper gegen die Behälterwand abgedichtet ist.

Um die Verweilzeit dokumentieren zu können, ist bei der Erfindung ein Positionsmesssystem zur Bestimmung der Position des Trennkörpers im Behälterhohlraum in Abhängigkeit von der Zeit vorgesehen. Hierdurch kann, vorzugsweise permanent, die Position des Trennkörpers im Behälterhohlraum bestimmt werden, womit auch dokumentiert werden kann, wie lange und wann sich wo, welches Teilvolumen des pumpfähigen Substrates im Behälter befindet bzw. befunden hat. In diesem Zusammenhang ist es günstig, wenn die Vorrichtung zumindest eine Temperaturmesseinrichtung zur Bestimmung der Temperatur des aus dem Behälter abströmenden pumpfähigen Substrats und/oder des auf den Behälter zuströmenden pumpfähigen Substrats aufweist. Hiermit kann nachgewiesen werden, dass das pumpfähige Substrat vor dem Eintritt in den Behälter bzw. das entsprechende Teilvolumen und beim Austritt aus dem entsprechenden Teilvolumen bzw. in den Behälter jeweils die benötigte Temperatur aufweist bzw. aufgewiesen hat.

Günstigerweise weist der Behälter auf jeder Seite des Trennkörpers zumindest einen Anschluss auf, durch den hindurch pumpfähiges Substrat in den Behälterhohlraum bzw. das auf der entsprechenden Seite des Trennkörpers liegende Teilvolumen des Behälterhohlraums eingefüllt und aus diesem auch wieder entnommen werden kann. In anderen Worten weist der Behälter günstigerweise auf einander entgegengesetzten Seiten des Trennkörpers entsprechende Anschlüsse auf, sodass in jedes Teilvolumen entsprechend pumpfähiges Substrat eingefüllt und aus diesem auch wieder entnommen werden kann.

Durch Verlagern, vorzugsweise Verschieben, des Trennkörpers im Behälterhohlraum verändern sich auch die Volumenanteile der Teilvolumina am Behälterhohlraum. Wird der Trennkörper in die eine Richtung verlagert, so nimmt das Teilvolumen auf der einen Seite ab und auf der anderen Seite zu und umgekehrt. (weiter auf Seite 4 der ursprünglichen Beschreibung)

Bevorzugte Ausgestaltungsformen sehen vor, dass der Trennkörper durch Einpumpen des pumpfähigen Substrats in eines der Teilvolumina unter gleichzeitigem Ablassen des pumpfähigen Substrats aus dem anderen der Teilvolumina innerhalb des Behälterhohlraums verlagert, vorzugsweise verschoben, wird. Bei einem entsprechenden Antrieb des Trennkörpers kann aber auch vorgesehen sein, dass der Trennkörper durch Verlagern bzw. Verschieben pumpfähiges Substrat in eines der Teilvolumina einsaugt und aus dem anderen herausdrückt und umgekehrt.

Erfindungsgemäße Vorrichtungen können sehr universell in sehr verschiedenen Einsatzbereichen eingesetzt werden. Entsprechend sind die Begriffe des Abtötens von Keimen und/oder Krankheitserregern breit auszulegen. Generell handelt es sich dabei um das Abtöten bzw. Beseitigen von Keimen und Krankheitserregern wie z.B. Bakterien, Viren, Pilzen, Hefen, anderen Mikroorganismen und dergleichen. Auch das pumpfähige Substrat, welches mit der erfindungsgemäßen Vorrichtung behandelt werden kann, kann sehr unterschiedlich zusammengesetzt sein. Es kann sich um eine Suspension, also um eine Mischung von Feststoffpartikeln und Flüssigkeiten und/oder um eine Emulsion, also eine Mischung von nicht ineinander lösbaren Flüssigkeiten handeln. Meist enthält das Substrat organische Anteile wie z.B. pflanzliche Abfälle, Speisereste, Reste aus der Tierverwertung, Fette, Öle und dergleichen. Es kann sich auch um ein zumindest teilweise vergorenes Substrat, Schlamm, Faulschlamm usw. handeln. In der Regel hat das pumpfähige Substrat einen Flüssigkeitsanteil, vorzugsweise einen Wasseranteil, um die Pumpfähigkeit herzustellen. Die Feststoffe sind entsprechend stark zerkleinert. Generell können erfindungsgemäße Vorrichtungen in der Abfallverwertung und Behandlung eingesetzt werden. Z.B. kann eine erfindungsgemäße Vorrichtung einer Biogasanlage vorgeschaltet und/oder nachgeschaltet und/oder in diese integriert sein, um im pumpfähigen Substrat Keime und/oder Krankheitserreger in dem gesetzlich vorgeschriebenen Umfang abzutöten.

Erfindungsgemäße Vorrichtungen können aber auch bei der Lebensmittelherstellung und -behandlung eingesetzt werden. So kann es sich beim pumpfähigen Substrat z.B. auch um Säfte, Milch oder andere pumpfähige Lebensmittel handeln. Die Behandlung des pumpfähigen Substrats im Behälterhohlraum kann mittels entsprechender Temperaturerhöhung oderabsenkung, also thermisch erfolgen. Es kann aber auch vorgesehen sein, dass die Behandlung chemisch erfolgt, indem man entsprechende chemische Substanzen zum pumpfähigen Substrat hinzugibt. Generell kann die erfindungsgemäße Vorrichtung überall dort eingesetzt werden, wo gewisse Verweilzeiten des pumpfähigen Substrats im Behälterhohlraum nötig und/oder gegebenenfalls gesetzlich vorgeschrieben sind.

Bei erfindungsgemäßen Vorrichtungen kann es sich z.B. um eine Vorrichtung zum Hygienisieren des pumpfähigen Substrates handeln. Beim Hygienisieren muss das pumpfähige Substrat für eine Verweilzeit von einer Stunde auf einer Temperatur von 70° C gehalten werden, um die entsprechenden Keime und/oder Krankheitserreger im gewünschten Maße abzutöten. Bei erfindungsgemäßen Vorrichtungen kann es sich aber auch um eine Vorrichtung zum Sterilisieren des pumpfähigen Substrates handeln. Hier muss das pumpfähige Substrat für eine Verweilzeit von zumindest 20 Minuten auf einer Temperatur von 133° C gehalten werden. Um noch ein anderes Beispiel zu nennen, kann eine erfindungsgemäße Vorrichtung auch zum Pasteurisieren, also zum kurzzeitigen Erhitzen des pumpfähigen Substrats auf 75° C oder 100° C eingesetzt werden.

Der Behälter der erfindungsgemäßen Vorrichtung, in dem sich der Behälterhohlraum zur Aufnahme des pumpfähigen Substrates befindet, könnte auch als Reaktor, Reaktorbehälter oder Behandlungsbehälter bezeichnet werden.

Für die Ausgestaltung des Trennkörpers gibt es verschiedene erfindungsgemäße Möglichkeiten. Eine erste davon sieht vor, dass der Trennkörper eine flexibel dehnbare Membran ist oder eine solche zumindest aufweist. Günstigerweise ist die Membran elastisch. Es kann vorgesehen sein, dass der gesamte Trennkörper aus einer einzigen Membran besteht. Es ist aber genauso gut möglich, dass der Trennkörper nur in Teilbereichen aus der Membran besteht und in anderen Teilbereichen aus einem Festkörperanteil wie z.B. einer Platte oder dergleichen. Die Membran besteht günstigerweise aus flexiblem, dauerhaft elastischem Material. Hier ist z.B. Kautschuk zu nennen. Insbesondere Butylkautschuke oder hydrierte Nitrilkautschuke sind in günstiger Art und Weise einsetzbar. Bevorzugt ist jedenfalls vorgesehen, dass die Membran in zumindest einem Befestigungsbereich der Membran ortsfest an der Behälterwand fixiert ist. Vorzugsweise handelt es sich dabei um einen am Rand der Membran liegenden Befestigungsbereich. Die außerhalb des Befestigungsbereiches liegenden Bereiche der Membran können dann entsprechend im Behälterhohlraum verlagert, vorzugsweise verschoben, werden.

In anderen Ausgestaltungsformen der Erfindung kann vorgesehen sein, dass der Trennkörper von der Behälterwand geführt aber ansonsten von der Behälterwand, vorzugsweise vom gesamten Behälter, losgelöst ist. In diesen Ausgestaltungsformen könnte man den Trennkörper auch als Stopfen bezeichnen, welcher innerhalb des Behälterhohlraums entsprechend verlagerbar, vorzugsweise verschiebbar, ist. Zur klaren und dauerhaften Abtrennung der Teilvolumina im Behälterhohlraum mittels des Trennkörpers ist bei diesen Ausgestaltungsformen günstigerweise vorgesehen, dass eine Außenkontur des Trennkörpers umfangsgeschlossen an einer den Behälterhohlraum umgebenden Innenfläche der Behälterwand anliegt. So kann der Trennkörper z. B. an seiner Außenkontur zumindest eine Dichtlippe und/oder zumindest eine Anordnung von Borsten zur Anlage an einer den Behälterhohlraum umgebenden Innenfläche der Behälterwand aufweisen. Sowohl die Dichtlippe als auch die Anordnung von Borsten sind günstigerweise umfangsgeschlossen ausgebildet, sodass die Dichtlippe bzw. die Borsten über den gesamten Umfang des Trennkörpers an der den Behälterhohlraum umgebenden Innenfläche der Behälterwand zur Anlage kommen.

Der Behälterhohlraum ist in bevorzugten Ausgestaltungsformen längserstreckt ausgeführt, d. h. seine Erstreckung in Längsrichtung ist größer als, vorzugsweise zumindest zwei- oder dreimal so groß wie, seine Quererstreckung bzw. wie sein Querschnitt. Besonders bevorzugt ist der Behälterhohlraum zumindest bereichsweise oder vollständig rohrförmig ausgebildet. Der Öffnungsquerschnitt des rohrförmigen Bereichs ist günstigerweise überall gleich bzw. konstant. In diesem Zusammenhang ist auch darauf hinzuweisen, dass der Behälter und damit auch der Behälterhohlraum liegend, stehend oder irgendwie anders ausgerichtet angeordnet werden können. Sie können gerade, gekrümmt oder auch einfach oder mehrfach umgelenkt ausgeführt werden. Die Längserstreckung ist unabhängig davon immer die Dimension der größten Erstreckung.

Bevorzugte Ausgestaltungsformen erfindungsgemäßer Vorrichtungen sehen vor, dass die Vorrichtung eine Heizeinrichtung zum Heizen des pumpfähigen Substrats aufweist. Diese Heizeinrichtung ist günstigerweise dem Behälter vorgelagert, sodass das pumpfähige Substrat bereits vor dem Eintritt in den Behälterhohlraum bzw. das entsprechende Teilvolumen auf die nötige Temperatur aufgeheizt ist. Grundsätzlich können hierzu verschiedenste, beim Stand der Technik an sich bekannte Heizeinrichtungen, wie z. B. auch Wärmetauscher eingesetzt werden. Günstig ist es z. B. wenn mittels der Heizeinrichtung Abwärme von Blockheizkraftwerken, Industrieanlagen o. dgl. zum Erwärmen bzw. Aufheizen des pumpfähigen Substrats genutzt wird. Es kann, gegebenenfalls auch zusätzlich zu einer anderen Heizeinrichtung, auch ein Wärmetauscher vorgesehen sein, der die Wärme des aus dem Behälterhohlraum abströmenden pumpfähigen Substrats zum Erwärmen bzw. Erhitzen des den Behälterhohlraum zuströmenden pumpfähigen Substrats nutzt.

Grundsätzlich ist es aber auch möglich, dass das Substrat im Behälterhohlraum selbst geheizt wird. Bevorzugt sehen solche Heizeinrichtungen aber dann im Wesentlichen vor, dass sie der Aufrechterhaltung der Temperatur des pumpfähigen Substrats im Behälterhohlraum bzw. dessen Teilvolumina dient. So kann z. B. eine wärmende und/oder thermisch dämmende Ummantelung für den Behälter vorgesehen sein. Es kann sich dabei z. B. um eine passive Dämmung aus Mineralwolle, Holzwolle, Glaswolle o. dgl. handeln, was an sich keine Heizung ist. Es kann sich aber auch um eine aktive Erwärmung des Behälters z. B. in Form eines Flüssigkeitsbades mit der entsprechenden Temperatur handeln. Diese wärmende und/oder thermisch dämmende Ummantelung kann auch direkt in die Behälterwand integriert sein oder diese entsprechend umgeben.

Ein erfindungsgemäßes Verfahren zum Betrieb einer erfindungsgemäßen Vorrichtung sieht jedenfalls vor, dass im Behälterhohlraum ein verlagerbarer vorzugsweise verschiebbarer, Trennkörper angeordnet ist, bzw. entsprechend verlagert oder verschoben wird, wobei der Trennkörper den Behälterhohlraum in zwei voneinander getrennte Teilvolumina zur Aufnahme des pumpfähigen Substrats trennt. Wie der Trennkörper im Behälterhohlraum verschoben bzw. verlagert werden kann, ist bereits weiter oben geschildert. Generell ist darauf hinzuweisen, dass sich aus den obigen Schilderungen zu den verschiedenen Varianten der Vorrichtung auch zahlreiche mögliche Details zu erfindungsgemäßen Ausgestaltungsformen eines entsprechenden Verfahrens ergeben.

Weitere Merkmale und Einzelheiten der vorliegenden Erfindung werden beispielhaft anhand von verschiedenen erfindungsgemäßen Ausführungsformen in der nachfolgenden kurzen und ausführlichen Figurenbeschreibung erläutert. Es zeigen:
Fig. 1 ein erstes erfindungsgemäßes Ausführungsbeispiel einer Vorrichtung mit zwei Behältern;
Fig. 2 ein zweites erfindungsgemäßes Ausführungsbeispiel einer Vorrichtung, diesmal mit drei Behältern;
die Fig. 3 bis 7 Darstellungen zu einer dritten erfindungsgemäßen Ausführungsform;
die Fig. 8 bis 10a vier weitere mögliche Ausgestaltungsformen erfindungsgemäßer Vorrichtungen;
die Fig. 11 und 12 drei verschiedene Ausgestaltungsformen von Trennkörpern, wie sie in den erfindungsgemäßen Vorrichtungen gemäß der Fig. 1 bis 10 eingesetzt werden können;
die Fig. 13 und 14 Schleusensysteme, welche in den Ausgestaltungsformen wie denen gemäß der Fig. 8 bis 10 eingesetzt werden können;
Fig. 15 eine erfindungsgemäße Vorrichtung mit einem Trennkörper in Form einer Membran;
Fig. 16 eine alternative Ausgestaltungsform eines Schleusensystems und
Fig. 17 eine Mischform der Schleusensysteme gemäß der Fig. 13, 14 und 16.

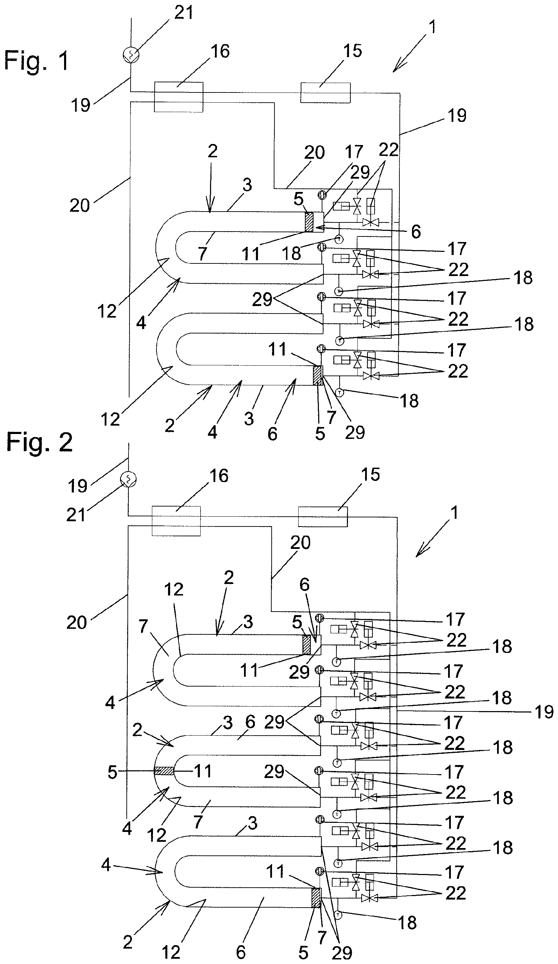

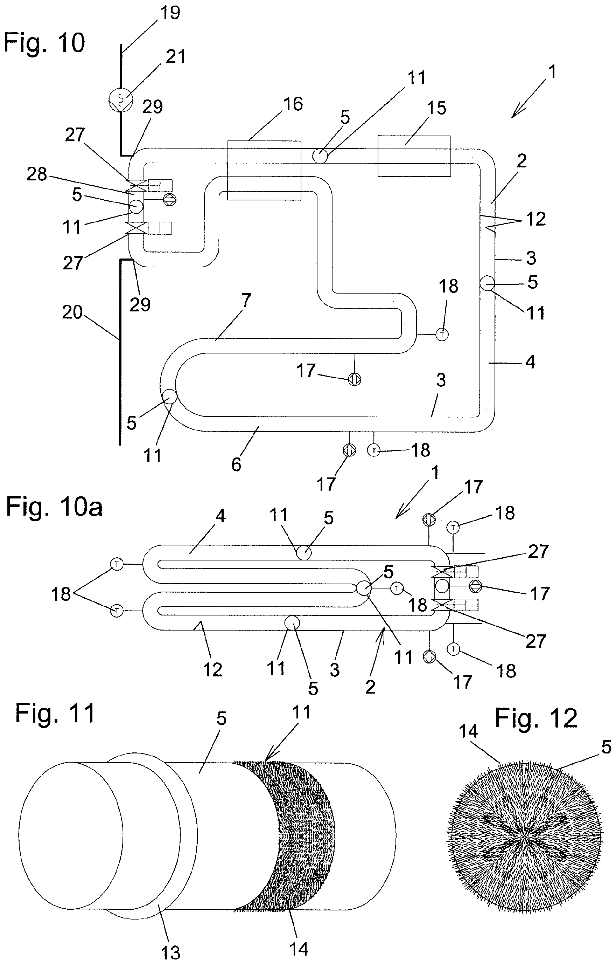

In der Ausgestaltungsform gemäß Fig. 1 einer erfindungsgemäßen Vorrichtung 1 wird das pumpfähige Substrat mittels der Pumpe 21 in der Zuführleitung 19 in Richtung hin zu den beiden Behältern 2 gepumpt. Im Wärmetauscher 16 wird die Wärme des in der Abführleitung 20 abgeführten, bereits fertig behandelten pumpfähigen Substrats zur Erwärmung des frischen, in der Zuführleitung 19 zugeführten pumpfähigen Substrats genutzt. Anschließend durchströmt das pumpfähige Substrat in der Zuführleitung 19 die Heizeinrichtung 15. Diese sorgt dafür, dass das pumpfähige Substrat auf das gewünschte Temperaturniveau gebracht wird. Hinter der Heizeinrichtung 15 führt die Zuführleitung 19 dann zu den beiden Behältern 2. Die Behälter 2 sind in diesem Ausführungsbeispiel rohrförmig und U-förmig gebogen ausgebildet. In ihnen ist erfindungsgemäß jeweils ein Trennkörper 5 verlagerbar, hier verschiebbar, angeordnet. Der Trennkörper 5 trennt den jeweiligen Behälterhohlraum 4 jeweils in zwei voneinander getrennte Teilvolumina 6 und 7, welche jeweils der Aufnahme des pumpfähigen Substrats dienen. Beim Trennkörper 5 handelt es sich hier um eine Art Stopfen, welcher innerhalb des Behälterhohlraums 4 verschiebbar gelagert ist. Dieser Stopfen kann, wie weiter hinten beschrieben und in Fig. 11 und 12 dargestellt, ausgeführt sein. Der Trennkörper 5 bzw. Stopfen in diesem Ausführungsbeispiel ist somit von der Behälterwand 3 des jeweiligen Behälters 2 geführt aber ansonsten von der Behälterwand 2 und auch vom gesamten Behälter 2 losgelöst. Die Außenkontur 11 des Trennkörpers 5 liegt umfangsgeschlossen an der den Behälterhohlraum 4 umgebenden Innenfläche 12 der Behälterwand 3 an. Günstigerweise ist dies so ausgeführt, dass der Trennkörper 5 gegen die Behälterwand 3 bzw. ihre Innenfläche 12 abgedichtet ist. Der Trennkörper 5 stellt eine Barriere zur Verhinderung der Vermischung von pumpfähigem Substrat aus einem der Teilvolumina 6 mit pumpfähigem Substrat aus dem anderen der Teilvolumina 7 dar. Beim Betrieb der erfindungsgemäßen Vorrichtung 1 gemäß Fig. 1 kann z. B. vorgesehen sein, dass durch Öffnen eines entsprechenden Ventils 22 das entsprechend erwärmte pumpfähige Substrat aus der Zuführleitung 19 über den Anschluss 29 in das Teilvolumen 6 des Behälterhohlraums 4 hineingepumpt wird. Gleichzeitig wird das entsprechende Ventil 22 geöffnet, sodass das pumpfähige Substrat aus dem Teilvolumen 7 dieses Behälters 2 in die Abführleitung 20 abströmen kann. Durch Hineinpumpen des pumpfähigen Substrats in das Teilvolumen 6 und Hinauslassen des pumpfähigen Substrats aus dem Teilvolumen 7 wird der Trennkörper 5 entlang des Behälterhohlraums 4 dieses Behälters 2 verschoben, bis er seine Endlage erreicht. Beim Hineinpumpen überwacht der entsprechende Temperatursensor 18, dass das in den Behälterhohlraum 4 hineingepumpte pumpfähige Substrat die gewünschte Temperatur besitzt. Das Erreichen der Endlage des Trennkörpers 5 wird durch den entsprechenden Positionsmesssensor 17 des Positionsmesssystems festgestellt. In diesem Zustand werden die entsprechenden Ventile 22 geschlossen. Das Teilvolumen 6 nimmt in dieser, in Fig. 1 in dem unteren Behälter 2 dargestellten Endlage nahezu den gesamten Behälterhohlraum 4 ein, während das Teilvolumen 7 auf ein Minimum reduziert ist. Mit dem Erreichen der entsprechenden Endlage durch den Trennkörper 5 beginnt nachweislich der Behandlungszeitraum, in dem sich das hier thermisch pumpfähige Substrat im Behälterhohlraum 4 auf dem gewünschten Temperaturniveau befindet. Erst wenn die gewünschte Verweildauer erreicht bzw. überschritten wird, wird dann durch entsprechendes Öffnen der entsprechenden Ventile 22 pumpfähiges Substrat aus der Zuführleitung 19 in das bisher auf das Minimum reduzierte Teilvolumen 7 gepumpt und bei entsprechendem Verschieben des Trennkörpers 5 entlang des Behälterhohlraums 4 wird das fertig behandelte pumpfähige Substrat durch entsprechende Reduzierung des Teilvolumens 6 aus dem Behälterhohlraum 4 über den entsprechenden Anschluss 29 in die Abführleitung 20 gedrückt. Dieser Vorgang wird dann so lange fortgesetzt, bis der Trennkörper 5 die entgegengesetzte Endlage am anderen Ende des Behälterholraums 2 erreicht hat, was wiederum mittels des dort angeordneten Positionsmesssensors 17 detektiert und überwacht werden kann. Beim Herausdrücken des fertig behandelten pumpfähigen Substrats aus dem Teilvolumen 6 misst der entsprechende, dort angeordnete Temperatursensor 18 die Temperatur im pumpfähigen Substrat bevor es in die Abführleitung 20 eingeführt wird und überwacht so, dass das pumpfähige Substrat auch nach der Verweilzeit noch auf dem benötigten Temperaturniveau ist. Hierdurch kann unter der erfindungsgemäßen Verwendung des Trennkörpers 5 überwacht und sichergestellt werden, dass das zu behandelnde pumpfähige Substrat für die gewünschte bzw. vorgeschriebene Verweildauer auf dem gewünschten bzw. vorgeschriebenen Temperaturniveau gehalten worden ist. Dies dokumentiert dann eindeutig, dass der vorgeschriebene bzw. gewünschte Prozess zum Abtöten der Keime und/oder Krankheitserreger im pumpfähigen Substrat eingehalten wurde. Durch die Verwendung von zwei oder mehr Behältern 2 in einer einzigen Vorrichtung 1 kann von einem intermittierenden auf einen kontinuierlichen Betrieb übergegangen werden. So kann z. B. das Befüllen und Entleeren der Teilvolumina 6 und 7 in einem der Behälter 2 zeitlich so abgestimmt sein, dass während dieser Zeit im zweiten Behälter 2 die gewünschten vorgeschriebenen Verweilzeiten des pumpfähigen Substrats in dem jeweiligen der Teilvolumina 6 und 7 des dortigen Behälterhohlraums 4 erreicht werden. Durch entsprechendes Umschalten zwischen den Behältern 2 kann so ein kontinuierlicher Eintrag und Austrag von pumpfähigem Substrat in und aus der Vorrichtung heraus ermöglicht werden.

Um sicherzustellen, dass das pumpfähige Substrat auch während seiner Verweilzeit im Behälterhohlraum 4 bzw. in den entsprechenden Teilvolumina 6 und 7 nicht unter das gewünschte Temperaturniveau abkühlt, können die Behälter 2 zusätzlich, wie eingangs bereits erläutert, mit einer wärmenden und/oder thermisch dämmenden Ummantelung versehen sein. Es kann sich z. B. um ein entsprechend aufgewärmtes Flüssigkeitsbad aber auch um eine gewöhnliche thermische Dämmung z. B. mit Mineralwolle o. dgl. handeln. Natürlich könnten auch aktive Heizeinrichtungen wie Glühstäbe, Heißdampfdüsen und dergleichen direkt in die Behälterwand 3 integriert sein.

Fig. 2 zeigt beispielhaft, dass bei einer Vorrichtung 1 gemäß Fig. 1 auch mehr als zwei Behälter 2 vorgesehen sein können. Ansonsten entspricht das Ausführungsbeispiel gemäß Fig. 2 in seinem grundsätzlichen Aufbau und seiner Funktion dem Ausführungsbeispiel gemäß Fig. 1, sodass eine zusätzliche Erläuterung dessen entfallen kann. Mehr als zwei Behälter 2 in einer Vorrichtung 1 bieten sich vor allem dann an, wenn größere Volumina an pumpfähigem Substrat, vorzugsweise kontinuierlich, behandelt werden sollen.

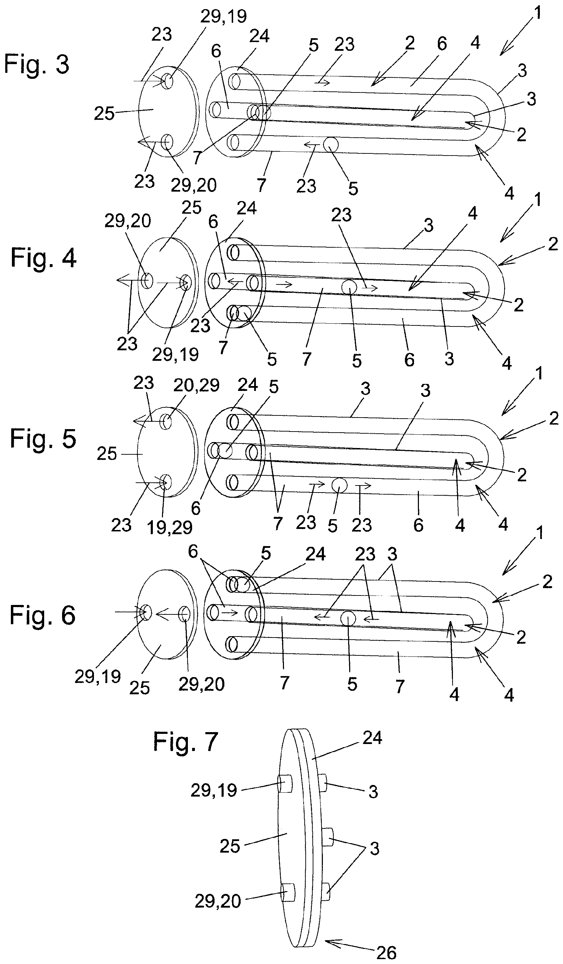

Die Fig. 3 bis 7 stellen schematisiert ein drittes erfindungsgemäßes Ausführungsbeispiel einer Vorrichtung 1 dar. Nicht gezeigt sind hier die Heizeinrichtung 15, der Wärmetauscher 16 sowie die Positionsmesssensoren 17 und die Temperaturmesseinrichtungen 18. Diese können aber in Anlehnung zu Fig. 1 ausgeführt sein. Die Zuführleitung 19 und die Abführleitung 20 für das jeweilige pumpfähige Substrat bzw. deren Anschlüsse 29 sind lediglich an der zweiten Drehventilplatte 25 des Drehventils 26 angedeutet.

Die Vorrichtung 1 dieses Ausführungsbeispiels weist zwei Behälter 2 auf. Diese sind wiederum als U-förmig gebogene Rohrleitungen ausgeführt. In jedem dieser Behälter 2 befindet sich im jeweiligen Behälterhohlraum 4 ein erfindungsgemäßer Trennkörper 5. Einer der Behälter 2 ist in einer vertikalen Ebene ausgerichtet. Der zweite Behälter 2 ist horizontal liegend ausgerichtet. In jedem der Behälterhohlräume 4 trennt der jeweilige Trennkörper 5 die Teilvolumina 6 und 7 voneinander ab. Es handelt sich auch hier beim Trennkörper 5 jeweils um eine Barriere zur Verhinderung der Vermischung von pumpfähigem Substrat aus einem der Teilvolumina 6 oder 7 mit pumpfähigem Substrat aus dem anderen der Teilvolumina 6 oder 7. Auch hier können wiederum Trennkörper 5 zum Einsatz kommen, wie sie beispielhaft in den Fig. 11 und 12 dargestellt sind. Man könnte auch hier bei den Trennkörpern 5 also von entsprechenden Stopfen sprechen. Das pumpfähige Substrat wird auch hier günstigerweise durch eine entsprechende, hier nicht dargestellte, Heizeinrichtung 15 und/oder einen entsprechenden Wärmetauscher 16 vor dem Einfüllen in das jeweilige Teilvolumen 6 oder 7 des jeweiligen Behälterhohlraums 4 auf die benötigte Temperatur aufgeheizt. Die Behälter 2 können zur Aufrechterhaltung der Temperatur in einer, hier ebenfalls nicht dargestellten, wärmenden und/oder thermisch dämmenden Ummantelung, wie z. B. einem entsprechenden Wärmebad gelagert sein.

Die Steuerung des Befüllens und Entleerens der Behälterhohlräume 4 bzw. der entsprechenden Teilvolumina 6 und 7 erfolgt hier über ein einziges Ventils in Form des Drehventils 26. Dieses in Fig. 7 schematisiert dargestellte Drehventil 26 weist eine erste Drehventilplatte 24 und eine zweite Drehventilplatte 25 auf, welche relativ zueinander verdreht werden können. Die erste Drehventilplatte 24 ist in diesem Ausführungsbeispiel ortsfest an die Behälterwände 3 der Behälter 2 angekoppelt. Die zweite Drehventilplatte 25 ist relativ dazu im und/oder entgegen des Uhrzeigersinns drehbar an der ersten Drehventilplatte 24 gelagert. An der zweiten Drehventilplatte 25 sind die entsprechenden Anschlüsse der Zuführleitung 19 und der Abführleitung 20 angeschlossen. Um das Verdrehen der zweiten Drehventilplatte 25 relativ zur ersten Drehventilplatte 24 nicht zu stören, können diese Zuführleitung 19 und Abführleitung 20 zumindest bereichsweise als Schläuche o. dgl. ausgeführt sein. Die relative Stellung zwischen zweiter Drehventilplatte 25 und erster Drehventilplatte 24 bestimmt jedenfalls ob und wenn ja, welche Teilvolumina 6 und 7 der jeweiligen Behälterhohlräume 4 mit pumpfähigem Substrat befüllt bzw. entsprechend entleert werden.

Günstigerweise sind entsprechende, hier nicht dargestellte Temperaturmesseinrichtungen 18 mehr oder weniger unmittelbar an der Mündung der Zuführleitung 19 und der Abführleitung 20 in die zweite Drehventilplatte 25 angeordnet, um die Temperatur des zuströmenden und abströmenden pumpfähigen Substrates überwachen zu können. Die hier ebenfalls nicht dargestellten Positionsmesssensoren 17 sind günstigerweise so angeordnet, dass sie jeweils detektieren können, wenn sich der jeweilige Trennkörper 5 in dem jeweiligen Behälterhohlraum 4 in einer seiner Endlagen in der Nähe der ersten Drehventilplatte 24 befindet.

Fig. 3 zeigt nun schematisiert den Vorgang des Befüllens des Teilvolumens 6 des vertikal stehenden Behälters 2. Die voneinander abgehobene Art der Darstellung der Drehventilplatten 24 und 25 dient rein der Veranschaulichung. In Fig. 3 wird das Drehventil 26 jedenfalls so geschaltet, dass aus der Zuführleitung 19 in Strömungsrichtung 23 pumpfähiges Substrat in das Teilvolumen 6 des Behälterhohlraums 4 des vertikal stehenden Behälters 2 eingepumpt wird. Hierdurch wird der Trennkörper 5 im Behälterhohlraum 4 des vertikal stehenden Behälters 2 verschoben und das pumpfähige Substrat aus dem Teilvolumen 7 des entsprechenden Behälterhohlraums 4 in die Abführleitung 20 gedrückt. Während dieses Vorgangs befindet sich das pumpförmige Substrat in den Teilvolumina 6 und 7 des Behälterhohlraums 4 des horizontal liegenden Behälters 2 in Ruhe. Der Trennkörper 5 dieses Behälters 2 befindet sich in einer seiner Endlagen, sodass das in dem dort vorhandenen Teilvolumen 6 vorhandene pumpfähige Substrat die gewünschte Verweilzeit zum Abtöten von Keimen und/oder Krankheitserregern erreichen kann. Die Dauer des Befüll- bzw. Entleervorgangs des Behälterhohlraums 4 des vertikal stehenden Behälters 2 entspricht günstigerweise der benötigten Verweilzeit, in der das pumpfähige Substrat im Teilvolumen 6 des horizontal liegenden Behälters 2 zur Abtötung der Keime und/oder Krankheitserreger ruht. Ist dieser Prozess gemäß Fig. 3 abgeschlossen und hat der Trennkörper 5 des vertikal stehenden Behälters 2 seine in Fig. 4 gezeigte Endlage erreicht, so wird das Drehventil 26 in die in Fig. 4 gezeigte Stellung gebracht, sodass die Zuführleitung 19 und die Abführleitung 20 nun mit den entsprechenden Teilvolumina 6 und 7 des Behälterhohlraums 4 des horizontal liegenden Behälters 2 in Verbindung gebracht werden. Nun beginnt das Befüllen des Teilvolumens 7 und das Entleeren des Teilvolumens 6 bei gleichzeitigem Verschieben des entsprechenden Trennkörpers 5 im Behälterhohlraum 4 des horizontal liegenden Behälters 2. In dieser Zeit verbringt das pumpfähige Substrat im Teilvolumen 6 des vertikal stehenden Behälters 2 die benötigte Verweilzeit in Ruhe. Auch hier sind die Strömungsrichtungen 23 eingezeichnet. Ist der Prozess gemäß Fig. 4 abgeschlossen, so wird das Drehventil 26 in die Stellung gemäß Fig. 5 gebracht. Hier gilt wieder entsprechendes. Ist der Prozess gemäß Fig. 5 abgeschlossen, so wird das Drehventil 26 in die Stellung gemäß Fig. 6 gebracht. Ist der dort stattfindende Prozess abgeschlossen, so beginnt der Zyklus wieder bei dem Vorgang gemäß Fig. 3. In allen vier Prozessvorgängen gemäß der Fig. 3 bis 6 ist jeweils sichergestellt, dass das pumpfähige Substrat in einem der Teilvolumina 6 oder 7 in einem der Behälter 2 zum Erreichen der gewünschten bzw. vorgeschriebenen Verweilzeit ruht, während die Teilvolumina 6 und 7 im jeweils anderen Behälter 2 in der Zwischenzeit befüllt bzw. entleert werden. Auch hierdurch ist ein mittels des Trennkörpers 5 sichergestellter und gut überwachbarer Prozess geschaffen, in dem die benötigten Verweilzeiten auf dem benötigten Temperaturniveau sichergestellt und überwacht werden können.

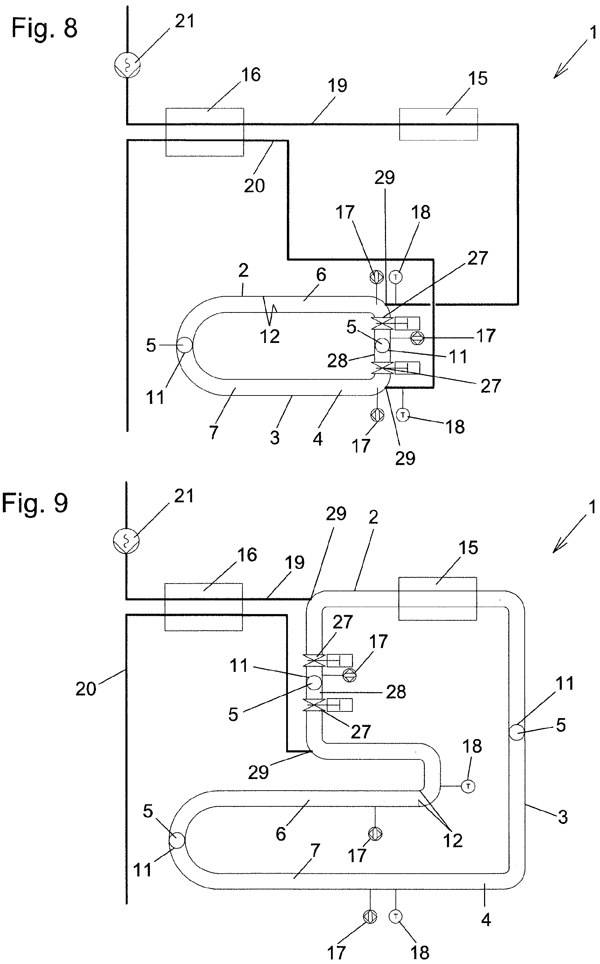

Während in den Ausführungsbeispielen gemäß der Fig. 1 bis 7 der Trennkörper 5 im Behälterhohlraum 4 hin und her bewegt wird, zeigen die Fig. 8 bis 10a Ausführungsbeispiele der Erfindung, bei denen ein oder mehrere Trennkörper 5 immer in ein und derselben Transportrichtung durch den Behälterhohlraum 4 transportiert wird bzw. werden. Dies ermöglicht es, dass der Behälterhohlraum 4 immer in derselben Richtung durchströmt wird. Fig. 8 zeigt ein erstes solches Ausführungsbeispiel, bei dem insgesamt zwei Trennkörper 5 im Behälterhohlraum 4 angeordnet sind. Das wiederum mittels der Pumpe 21 in der Zuführleitung 19 zugeführte und mittels des Wärmetauschers 16 und der Heizeinrichtung 15 auf die benötigte Temperatur erwärmte pumpfähige Substrat wird nach Passieren der Temperaturmesseinrichtung 18 über den entsprechenden Anschluss 29 in das Teilvolumen 6 des Behälterhohlraums 4 des Behälters 2 gemäß Fig. 8 eingefüllt. Gleichzeitig wird das im Teilvolumen 7 sich befindliche pumpfähige Substrat über den entsprechenden Anschluss 29, die Abführleitung 20 und damit auch den Wärmetauscher 16 aus dem Behälterhohlraum 4 hinausgedrückt. Der Trennkörper 5, welcher ebenfalls z. B. in einer der in Fig. 11 und 12 gezeigten Ausführungsformen ausgestaltet sein kann, wird dabei wiederum innerhalb des Behälterhohlraums 4 verlagert bzw. verschoben. Ist das Teilvolumen 6 vollständig gefüllt und das Teilvolumen 7 im Wesentlichen geleert, so erreicht der Trennkörper 5 den in Fig. 8 unten dargestellten Positionsmesssensor 17 des Positionsmesssystems. Hat der Trennkörper 5 diesen Punkt am unteren Positionsmesssensor 17 erreicht, so kann die Pumpe 21 ausgestellt werden und das in das Teilvolumen 6 eingepumpte pumpfähige Substrat verbleibt die benötigte Verweilzeit im Behälterhohlraum 4. Ist die benötigte Verweilzeit verstrichen, so kann mittels des durch die Schieber 27 gebildeten Schleusensystems ein bisher im Zwischenbereich 28 zwischen den beiden Schiebern 27 in Warteposition gehaltener Trennkörper 5 in den Bereich des in Fig. 8 oben dargestellten Positionsmesssensors 17, also in den Bereich der Mündung der Zuführleitung 19 in den Behälterhohlraum 4 entlassen bzw. eingeschleust werden. Anschließend wird der Trennkörper 5, welcher bisher am unteren Positionsmesssensor 17 verharrt hat, in den Zwischenbereich 28 zwischen die Schieber 27 aufgenommen. Sind anschließend daran beide Schieber 27 wieder geschlossen, so kann die Pumpe 21 wieder angestellt werden und der dann dort vorhandene Trennkörper 5 wird durch Einpumpen von pumpfähigem Substrat in das Teilvolumen 7 wieder langsam Richtung unterem Positionsmesssensor 17 durch den Behälterhohlraum 4 geschoben. Dabei wird nun das Teilvolumen 6 in Bewegungsrichtung vor dem Trennkörper 5, in dem sich nun ausreichend lang wärmebehandeltes pumpfähiges Substrat befindet, in die Abführleitung 20 hinausgedrückt. Der untere, an der Abführleitung 20 vorhandene Temperatursensor 18 überwacht dabei, dass sich das pumpfähige Substrat noch auf dem gewünschten Temperaturniveau befindet. Auch die in den Fig. 8 bis 10a gezeigten Behälter 2 können durch eine entsprechende thermisch dämmende oder wärmende Ummantelung zusätzlich geheizt bzw. vor Auskühlung geschützt werden. Auch dies gilt für alle vier Ausführungsbeispiele gemäß der Fig. 8, 9, 10 und 10a.

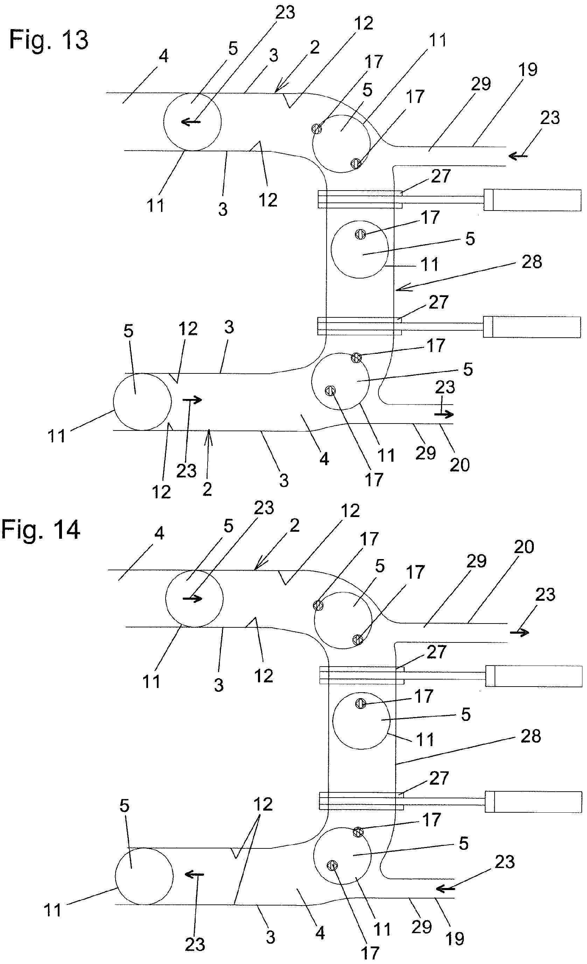

Die Funktionsweise des durch die Schieber 27 gebildeten Schleusensystems wird anhand der Fig. 13 erläutert. Wichtig ist zunächst in diesem Zusammenhang darauf hinzuweisen, dass für diese Ausführungsvariante der Trennkörper 5 eine geringere Dichte als das ihn umgebende pumpfähige Substrat aufweisen sollte.

In Fig. 13 sind mehrere Positionen des Trennkörpers 5 jeweils als Trennkörper dargestellt. Der Ablauf des Schleusens eines Trennkörpers 5 kann wie folgt durchgeführt werden: Zunächst bewegt sich der unten in Fig. 13 dargestellte Trennkörper 5 entlang des Behälterhohlraums 4 in Strömungsrichtung 23 bis er die unteren beiden Positionsmesssensoren 17 erreicht. Ein zweiter Trennkörper ist in dieser Zeit zwischen den beiden geschlossenen Schiebern 27 im Zwischenbereich 28 gefangen. Ist der untere Trennkörper 5 nun bei den unteren Positionsmesssensoren 17 angelangt, so bleibt er dort zunächst bei geschlossenem unterem Schieber 27 stehen. Der in Fig. 13 oben dargestellte Schieber 27 wird geöffnet. Der bislang im Zwischenbereich 28 zurückgehaltene Trennkörper 5 kann nun aufgrund seiner, gegenüber dem ihm umgebenden pumpfähigen Substrat geringeren Dichte durch den geöffneten oberen Schieber 27 hindurch aufschwimmen und gelangt in den Bereich der Mündung der Zuführleitung 19 in den Behälterhohlraum 4 und damit in den Bereich der oberen beiden Positionsmesssensoren 17. Stellen diese fest, dass nun ein Trennkörper 5 bei ihnen angelangt ist, so wird der obere Schieber 27 geschlossen und der untere Schieber 27, wenn die benötigten Verweilzeiten erreicht sind, geöffnet. Nun steigt der bisher an den unteren Positionsmesssensoren 17 in Wartestellung verbliebene Trennkörper 5 aufgrund seiner geringen Dichte in dem ihn umgebenden pumpfähigen Substrat auf. Er gelangt hierdurch in den Zwischenbereich 28 und wird dort von dem dort angeordneten mittleren Positionsmesssensor 17 erkannt. Sobald dies der Fall ist, wird der untere Schieber 27 wieder geschlossen. Ist dieser Zustand dann erreicht, so kann, wie vorab bereits geschildert, wieder frisches pumpfähiges Substrat über die Zuführleitung 19 in den Behälterhohlraum 4 eingepumpt werden, sodass dann der an den oberen Sensoren 17 wartende Trennkörper 5 in Richtung 23 geschoben wird, wobei gleichzeitig das Teilvolumen in Richtung 23 hinter dem Trennkörper 5 gefüllt und das pumpfähige Substrat aus dem Teilvolumen in Richtung 23 vor dem Trennkörper 5 über die Abführleitung 20 aus dem Behälterhohlraum 4 herausgedrückt wird.

Der Vollständigkeit halber ist noch darauf hinzuweisen, dass im Zwischenbereich 28 und in den Bereichen der Krümmung vor und hinter dem Zwischenbereich 28 der Behälterhohlraum 4 ein gegenüber dem restlichen Behälterhohlraum 4 aufgeweiteten Querschnitt aufweist, wie dies in Fig. 13 auch gut zu sehen ist. Hierdurch wird es möglich, dass pumpfähiges Substrat in diesem Zwischenbereich und den daran angrenzenden gekrümmten Bereichen am Trennkörper 5 vorbeiströmt, was zum Aufstieg des Trennkörpers 5 ja auch notwendig ist. In den anderen Bereichen des Behälterhohlraums 4 liegt der Trennkörper 5 jeweils mit seiner Außenkontur 11, wie bereits besprochen, umfangsgeschlossen an der den Behälterhohlraum 4 umgebenden Innenfläche 12 der Behälterwand 3 an. Dies ist beispielhaft in Fig. 13 bei den links dargestellten oben und unten im Behälterhohlraum 4 angeordneten Trennkörpern 5 gezeigt. In diesen Bereichen bildet der Trennkörper 5 somit eine Barriere zur Verhinderung der Vermischung von pumpfähigem Substrat aus dem einen der Teilvolumina 6 mit pumpfähigem Substrat aus dem anderen der Teilvolumina 7.

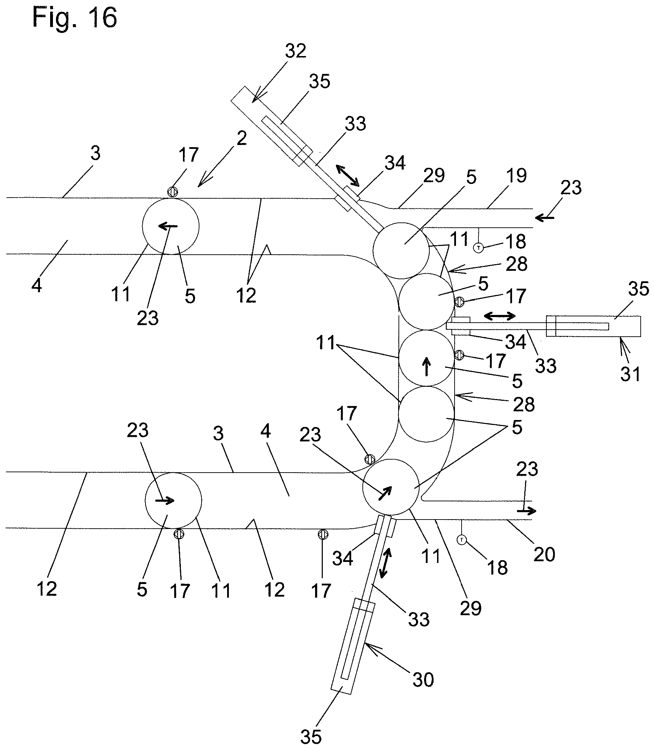

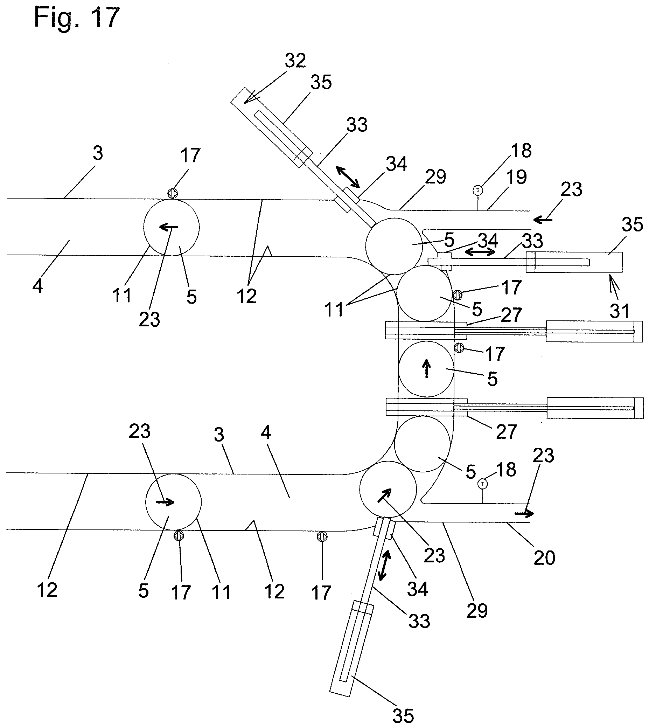

Fig. 14 zeigt eine alternative Variante zu der Schleuse gemäß Fig. 13, welche dann eingesetzt werden kann, wenn der Trennkörper 5 eine größere Dichte als das ihn umgebende pumpfähige Substrat aufweist. Am Rohrleitungssystem ändert sich konstruktiv dabei nur, dass die Mündungsstellen von Zuführleitung 19 und Abführleitung 20 vertauscht sind, wie dies in Fig. 14 durch die Strömungsrichtungspfeile 23 dargestellt und auch entsprechend mittels der Bezugszeichen 19 und 20 markiert ist. Bei dieser Variante gemäß Fig. 14 kommt der Trennkörper 5 in Bewegungsrichtung 23 an den oberen beiden Positionsmesssensoren 17 an. Dies ist das Signal zum Abschalten der Pumpe 21 und zum Öffnen des in Fig. 14 unten dargestellten Schiebers 27. Hierdurch kann der zweite bislang im Zwischenbereich 28 zurückgehaltene Trennkörper 5 durch den unteren geöffneten unteren Schieber 27 hindurch in den Bereich vor der Mündung der Zuführleitung 19 absinken. Dies geschieht bei diesen Varianten aufgrund der größeren Dichte des Trennkörpers 5 verglichen mit dem ihm umgebenden pumpfähigen Substrat. Hat dieser aus dem Zwischenbereich 28 nun entlassene Trennkörper 5 die beiden unteren Positionsmesssensoren 17 erreicht, so schließt der untere Schieber 27 und anschließend öffnet der obere Schieber 27, sodass der an den oberen Positionsmesssensoren erwartete Trennkörper 5 bei geöffnetem oberem Schieber 27 in den Zwischenbereich 28 absinken kann. Auch dies erfolgt aufgrund der größeren Dichte des Trennkörpers 5 verglichen mit dem ihn umgebenden pumpfähigen Substrat. Erreicht dieser bislang obere Trennkörper 5 den Positionsmesssensor 17 im Zwischenbereich 28, so wird der obere Schieber 27 geschlossen und der Pumpvorgang des Einpumpens von frischem pumpfähigem Substrat über die Zuführleitung 19 in den Behälterhohlraum 4 kann wieder beginnen. Durch die in den Fig. 13 und 14 und auch in den weiter unten noch erläuterten Fig. 16 und 17 gezeigten Schleusensysteme wird verhindert, dass über die Zuführleitung 19 frisch eingefülltes pumpfähiges Substrat mit bereits fertig behandeltem über die Abführleitung 20 abgepumptem pumpfähigem Substrat vermischt wird und ermöglicht, dass der bzw. die Trennkörper 5 immer in derselben Richtung durch den jeweiligen Behälterhohlraum 4 transportiert werden. Das Substrat bei dem die Keime und/oder Krankheitserreger ausreichend abgetötet wurden, wird also nicht mehr kontaminiert. Die Vorgänge des Schleusens der Trennkörper 5 sind zeitlich entsprechend der benötigten Verweildauern zum Abtöten der Keime und/oder Krankheitserreger im pumpfähigen Substrat durchzuführen.

In den Fig. 13 und 14 sind jeweils zwei obere und untere Positionsmesssensoren 17 oberhalb und unterhalb der Schieber 27 eingezeichnet. Man kommt aber grundsätzlich an diesen Stellen auch mit jeweils einem Positionsmesssensor 17 aus, wie dies jeweils vereinfacht in den Fig. 8 bis 10a dargestellt ist.

Die Schleusensysteme gemäß den Fig. 13, 14, 16 und 17 können jedenfalls bei allen vier Ausführungsbeispielen gemäß der Fig. 8 bis 10a eingesetzt werden, wobei dann eben jeweils an den richtigen Stellen die Zuführleitung 19 und die Abführleitung 20 in den Behälterhohlraum 4 einmünden müssen.

Die Ausführungsvarianten gemäß der Fig. 9,10 und 10a entsprechen in ihrem Grundaufbau dem Ausführungsbeispiel gemäß Fig. 8. In Fig. 9 ist allerdings der Behälter 2 so groß bzw. lang ausgeführt, dass die Heizung 15 im Bereich des Behälters 2 angeordnet ist. In Fig. 10 ist das System so groß erweitert, dass auch der Wärmetauscher 16 im Bereich des Behälters 2 auf das pumpfähige Substrat einwirkt. Bei allen drei Ausführungsvarianten gemäß den Fig. 9, 10 und 10a sind mehrere voneinander beabstandete Trennkörper 5 im Behälterhohlraum 4 hintereinander angeordnet. Der Abstand zwischen zwei hintereinander angeordneten Trennkörpern 5 sollte dem Abstand zwischen den zwei jeweils unten dargestellten Positionsmesssensoren 17 entsprechen, sodass wenn ein Trennkörper 5 den einen dieser beiden unteren Positionsmesssensoren 17 passiert, der darauffolgende Trennkörper 5 den anderen dieser beiden unteren Positionsmesssensoren 17 passiert. Der Abschnitt des Behälterhohlraums 4 zwischen diesen beiden Positionsmesssensoren 17 wird somit in diesem Fall als Messstrecke verwendet, um die benötigten Verweilzeiten zu kontrollieren. Die Temperatur des pumpfähigen Substrats im Behälterhohlraum 4 wird jeweils durch die beiden Temperaturmesseinrichtungen 18 vor und hinter dieser Messstrecke überwacht. Auch durch diese Varianten kann überwacht werden, dass die Verweilzeit des pumpfähigen Substrates auf dem benötigten Temperaturniveau eingehalten wird.

In Fig. 10a sind nur Teile der Anlage gemäß dieses Ausführungsbeispiels dargestellt.

Der nicht dargestellte Rest, also die Zuführleitung 19, die Abführleitung 20, die Pumpe 21 sowie Wärmetauscher 16 und Heizeinrichtung 15 usw. können wie bei Fig. 8 ausgeführt sein. Fig. 10a veranschaulicht beispielhaft, dass der Behälter 2 und damit auch der Behälterhohlraum 4 auch mehrfach umgelenkt ausgeführt sein können. Weiters wird beispielhaft gezeigt, dass mittels entsprechender Temperaturmesseinrichtungen 18 die Temperatur des pumpfähigen Substrats auch innerhalb des Behälterhohlraums 4 bzw. auch innerhalb der oben genannten Messstrecken überwacht bzw. gemessen werden kann. Dies kann, auch wenn dies bei den anderen Ausführungsbeispielen nicht explizit dargestellt ist, bei allen Ausführungsvarianten der Erfindung so realisiert werden.

In den Fig. 11 und 12 sind nun beispielhaft Trennkörper 5 in Form von Stopfen gezeigt, welche für die bislang beschriebenen Ausführungsbeispiele geeignet sind. Die Außenkontur 11 der Trennkörper 5 in den Fig. 11 und 12 ist so auf den Querschnitt des Behälterhohlraums 4 abgestimmt, dass die Außenkontur 11 des Trennkörpers 5 umfangsgeschlossen an der den Behälterhohlraum 4 umgebenden Innenfläche 12 der Behälterwand anliegt. Somit ist klar, dass bei anderen Querschnittsflächen des Behälterhohlraums 4 auch andere Außenkonturen 11 der jeweiligen Trennkörper 5 vorgesehen sein sollten.

In Fig. 11 ist der Trennkörper 5 jeweils jedenfalls als Zylinder ausgeführt. An seiner Außenkontur sind beispielhaft eine umfangsgeschlossene Dichtlippe 13 und eine Anordnung von Borsten 14 angeordnet. Es können natürlich an einem entsprechenden Trennkörper 5 auch nur Dichtlippen 13 oder nur Anordnungen von Borsten 14 angeordnet sein. In Fig. 12 ist der Trennkörper 5 eine Kugel, dessen gesamte Außenkontur 11 mit einer Anordnung von Borsten 14 besetzt ist. Sowohl mittels der Dichtlippe 13 als auch mit einer entsprechend dichten Ausgestaltung der Anordnung der Borsten 14 kann eine Abdichtung des Trennkörpers 5 gegen die Behälterwand 3 sichergestellt werden. Außerdem bewirken die Borsten 14 wie auch die Dichtlippe 13 eine Reinigung der Innenfläche 12 der Behälterwand 3 wenn der Trennkörper 5 durch den Behälterhohlraum 4 hindurchgeschoben wird. Unabhängig davon, welche Form der Trennkörper 5 nun hat, ist bevorzugt vorgesehen, dass der Trennkörper ein Festkörper ist, der die notwendige Steifigkeit und an die Innenfläche 12 der Behälterwand 3 angepasste Außenkontur 11 aufweist, um auf seinem gesamten Weg durch den Behälterhohlraum 4 eine entsprechende Barriere zur Verhinderung der Vermischung von pumpfähigem Substrat aus den Teilvolumina 6 und 7 vor und hinter dem Trennkörper 5 zu bilden. Bei den Trennkörpern 5 kann es sich z.B. um Hohlkörper handeln. Die Länge der Borsten 14 kann z.B. bei 7 bis 10mm (Millimeter) liegen. Die Borsten 14 könnten abweichend von Fig. 12 auch nur auf Teilbereichen der Oberfläche des Trennkörpers 5 angeordnet sein. Z.B. wäre es bei kugelförmigen Trennkörpern 5 auch möglich, nur auf Schnittpunkten von gedachten Längen- und Breitenkreisen auf der Oberfläche der Kugel jeweils Anordnungen von Borsten 14 anzuordnen. Die Trennkörper 5 könnten auch aus Halbschalen gefertigt und dann entsprechend zusammengefügt werden. Die Trennkörper 5 sollten aber jedenfalls dauerhaft die jeweils benötigte Temperaturfestigkeit aufweisen.

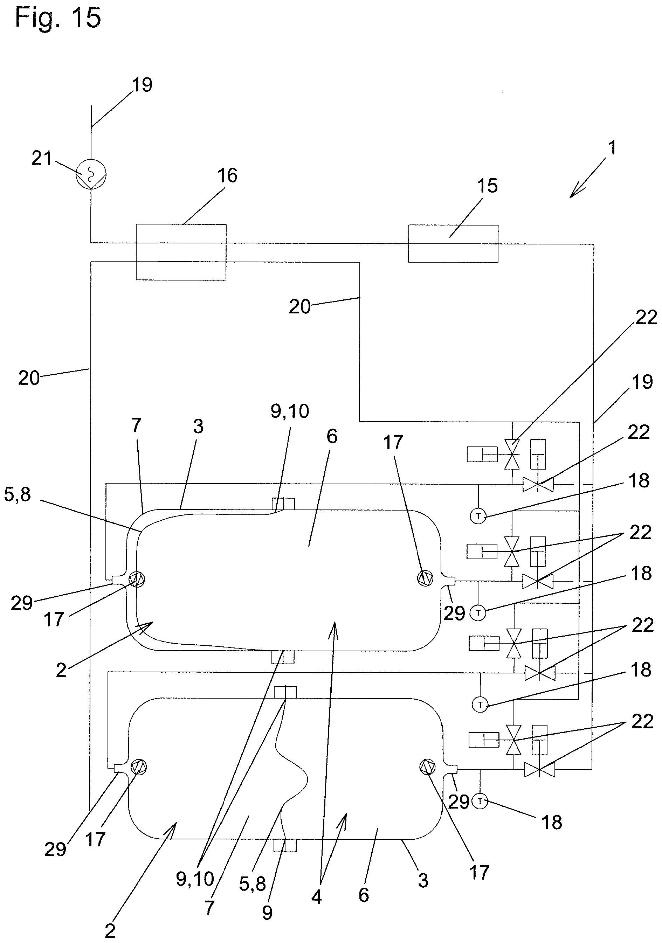

Im Gegensatz zu den bisher diskutierten Ausführungsbeispielen der Erfindung ist in der Ausführungsvariante gemäß Fig. 15 nun ein Trennkörper 5 in Form einer dehnbaren Membran 8 vorgesehen. Auch dieser Trennkörper 5 befindet sich zwischen zwei Teilvolumina 6 und 7 des Behälterhohlraums 4. Durch entsprechende Deformation der Membran 8 ist auch dieser Trennkörper 5 im Behälterhohlraum 4 so verlagerbar bzw. außerhalb seines Befestigungsbereichs 10 auch verschiebbar, dass sich die Volumenanteile der Teilvolumina 6 und 7 am Behälterhohlraum 4 entsprechend verändern. Auch diese Art des Trennkörpers 5 stellt eine Barriere zur Verhinderung der Vermischung von pumpfähigem Substrat aus einem der Teilvolumina 6 mit pumpfähigem Substrat aus dem anderen der Teilvolumina 7 dar. Im gezeigten Ausführungsbeispiel gemäß Fig. 15 weist die Vorrichtung 1 zwei im Wesentlichen baugleiche Behälter 2 auf, um einen konstanten Betrieb der Vorrichtung 1 mit konstanter Abgabe und Zufuhr von pumpfähigem Substrat zu gewährleisten. In beiden Behältern 2 ist die jeweilige Membran 8 mit einem am Rand 9 der Membran 8 liegenden Befestigungsbereich 10 ortsfest an der Behälterwand 3 fixiert. Es bietet sich an, dass diese Fixierung mittig zwischen den Anschlüssen 29 angeordnet ist, an denen die Zuführleitung 19 und die Abführleitung 20 in den Behälterhohlraum 4 münden.

An den entsprechenden Enden in der Nähe der Anschlüsse 29 befinden sich jeweils Positionsmesssensoren 17, welche feststellen können, ob die Membran 8 und damit der Trennkörper 5 in ihre Nähe verlagert ist oder nicht. Vor und hinter jedem Anschluss 29 befindet sich auch wiederum jeweils eine Temperaturmesseinrichtung 18, mit welcher die Temperatur des in den Behälterhohlraum 4 ein- bzw. ausströmenden pumpfähigen Substrats gemessen werden kann. Auch die in der Fig. 15 gezeigten Behälter 2 können von einer wärmenden oder zumindest thermisch dämmenden Ummantelung umgeben sein, welche hier wiederum nicht dargestellt ist.

Auch in dieser Variante gemäß Fig. 15 wird das frische pumpfähige Substrat in der Zuführleitung 19 mittels der Pumpe 21 gepumpt und zunächst mittels des Wärmetauschers 16 und der Heizeinrichtung 15 auf das benötigte Temperaturniveau gebracht. Anschließend wird es in einen der beiden Behälter 2 gefüllt. In der Darstellung gemäß Fig. 15 ist im weiter oben dargestellten Behälter 2 das Teilvolumen 6 des Behälterhohlraums 4 so weit gefüllt, dass die Membran 8 also der Trennkörper 5 maximal ausgelenkt ist. Mittels des entsprechenden Positionsmesssensors 17 kann überwacht werden, dass die Membran 8 diese Position hält, in der das Teilvolumen 7 dieses Behälterhohlraums 4 im Wesentlichen entleert ist. In diesem Zustand wird bei entsprechend geschlossenen Ventilen 22 dieser Behälter 2 belassen, bis die zum Abtöten der Keime und/oder Krankheitserreger benötigte bzw. vorgeschriebene Verweilzeit erreicht ist. In dieser Zeit wird der andere, in der in Fig. 15 gezeigten Momentaufnahme der untere der Behälter 2 frisch befüllt. Es wird also über die Zuführleitung 19 ein entsprechend auf Temperatur gebrachtes pumpfähiges Substrat in das Teilvolumen 6 eingepumpt, womit durch entsprechende Verlagerung der Membran 8 das gegenüberliegende Teilvolumen 7 dieses Behälters 2 entleert und damit das dort sich befindliche ausreichend wärmebehandelte pumpfähige Substrat aus dem Teilvolumen 7 in die Abführleitung 20 gedrückt wird. Der Vorgang des Befüllens dieses Teilvolumens 6 und des Ausleerens des Teilvolumens 7 dauert vorzugsweise genauso lange, wie die Verweilzeit des pumpfähigen Substrats im Teilvolumen 6 oder 7 des jeweils anderen Behälters 2 dauert. Wird dann im unteren Behälter bei Erreichen der Position des Positionsmesssensors 17 durch die Membran 8 der Einfüll- und Ausdrückvorgang in diesem Behälter 2 beendet, so kehrt sich die Vorgehensweise um und der frisch befüllte Behälter 2 verbleibt während der Verweilzeit unverändert während der andere Behälter 2 wiederum in einem Teilvolumen entleert und im anderen Teilvolumen befüllt wird. Bei dieser Betriebsweise kann also kontinuierlich pumpfähiges Substrat dem System in der Zuführleitung 19 zugeführt und in der Abführleitung 20 entnommen werden.

Werden größere Volumina gefordert, so können auch mehrere Behälter 2 entsprechend in der Vorrichtung 1 verwendet werden. Auch der Betrieb mit nur einem Behälter 2 ist denkbar, wobei dann eben eine intermittierende Förderung und thermische Behandlung des pumpfähigen Substrates erfolgt.

In Fig. 16 ist noch eine weitere, alternativ zu den Varianten gemäß der Fig. 13 und 14 mögliche, Ausgestaltungsform eines Schleusensystems für die Trennkörper 5 gezeigt. In dieser Variante wird auf die Schieber 27 der beiden Varianten gemäß Fig. 13 und 14 zum Abschließen des Zwischenbereichs 28 verzichtet. Ein weiterer Unterschied liegt darin, dass es bei der Variante gemäß Fig. 16 im Bereich des Schleusensystems keine Aufweitung des Querschnitts des Behälterhohlraums 4 gibt. Dieser hat in der Variante gemäß Fig. 16 im Bereich des Schleusensystems denselben Öffnungsquerschnitt wie außerhalb des Schleusensystems. Hierdurch kann die Schleuse dadurch verschlossen werden, dass man zumindest einen der Trennkörper 5 im Zwischenbereich 28 in seiner Position festhält. Zum Festhalten des Trennkörpers 5 ist im in Fig. 16 schematisch dargestellten Ausführungsbeispiel die Rückhaltesperre 31 vorgesehen. Diese weist zumindest zwei Stellungen auf. In der einen Stellung kann ein Trennkörper 5 die Rückhaltesperre 31 passieren. In einer anderen Stellung wird der Trennkörper 5 von der Rückhaltesperre 31 in seiner Position festgehalten. Zusätzlich zu der Rückhaltesperre 31 ist noch eine Schubvorrichtung 30 vorhanden, welche die in Strömungsrichtung 23 vor dem Zwischenbereich 28 ankommenden Trennkörper 5 in den Zwischenbereich 28 aktiv hineinschieben kann. Weiters kann diese Schubvorrichtung 30 bei entsprechend geöffneter Rückhaltesperre 31 auch dazu verwendet werden, einen Trennkörper 5 in dem in Strömungsrichtung 23 gesehen, hinter den Zwischenbereich 28 liegenden Bereich, wieder über den Anschluss 29 der Zuführleitung 19 hinauszuschieben. Im gezeigten Ausführungsbeispiel gemäß Fig. 16 wird dies so bewerkstelligt, dass sich im Zwischenbereich 28 mehrere Trennkörper 5 hintereinander liegend sammeln, sodass durch Schieben des in Strömungsrichtung 23 vor dem Zwischenbereich 28 liegenden Trennkörpers 5 der in Strömungsrichtung 23 hinter dem Zwischenbereich 28 liegende Trennkörper 5 auch weitergeschoben wird, wenn die Rückhaltesperre 31 entsprechend geöffnet ist.

Optional kann bei dieser Variante noch die zusätzliche Rückhaltesperre 32 vorhanden sein. Diese kann, soweit notwendig, dazu genutzt werden, zu verhindern, dass Trennkörper 5 ungewollt den Anschluss 29 der Zuführleitung 19 blockieren. Im gezeigten Ausführungsbeispiel sind die Schubvorrichtung 30, die Rückhaltesperre 31 und die zusätzliche Rückhaltesperre 32 jeweils identisch ausgebildet. Sie weisen jeweils eine Stange 33 auf, die durch eine entsprechend abgedichtete Durchführung 34, wie z.B. einen Balg oder dergleichen, durch die Behälterwand 3 hindurch in den Behälterhohlraum 4 hineingeschoben werden können. Die Durchführungen 34 sind hier nur schematisiert dargestellt, in Realität aber so ausgeführt, dass die Trennkörper 5 an den Durchführungen 34 vorbei bewegt werden können, ohne mit ihnen zu kollidieren. Jeder dieser Stangen 33 ist ein Antrieb 35 zugeordnet, mit dem die jeweilige Stange 33 in ihrer Längsrichtung vor- und zurückbewegt werden, also in den Behälterhohlraum 4 hineingeschoben und aus diesem herausgezogen werden kann. Die Antriebe 35 können in an sich bekannter Art z.B. pneumatisch, hydraulisch, elektrisch oder dergleichen ausgebildet sein. Dies ist im Stand der Technik ausreichend bekannt und muss nicht weiter erläutert werden. Alternativ könnten die Schubvorrichtung 30, die Rückhaltesperre 31 und/oder die zusätzliche Rückhaltesperre 32 auch als Spindelgetriebe, wobei die Spindeln dann die Stange 33 bilden, ausgebildet sein.

Bei dieser Variante gemäß Fig. 16 muss aufgrund der Verwendung der Schubvorrichtung 30 und der Rückhaltesperre 31 sowie gegebenenfalls der zusätzlichen Rückhaltesperre 32 kein Dichteunterschied zwischen dem Trennkörper 5 und dem pumpfähigen Substrat im Behälterhohlraum 4 vorhanden sein, um die Trennkörper 5 durch den Zwischenbereich 28 hindurchzubewegen. Ob ein solcher Dichteunterschied vorhanden ist oder nicht, ist in diesem Ausführungsbeispiel egal.

In der in Fig. 16 gezeigten Variante sind zahlreiche Positionsmesssensoren 17 an unterschiedlichen Stellen angeordneten. Es sind auch Varianten möglich, die mit mehr oder weniger Positionsmesssensoren 17 ausgestattet sein können.

Bei der in Fig. 17 gezeigten Ausgestaltungsform eines Schleusensystems für die Trennkörper 5 handelt es sich um eine Mischform der Varianten gemäß der Fig. 13 und 14 einerseits und gemäß der Fig. 16 andererseits. Erläutert werden hier nur noch die Unterschiede zu der Variante gemäß Fig. 16, ansonsten kann die Variante gemäß Fig. 17, wie bezüglich Fig. 16 beschrieben, ausgeführt und betrieben werden. Diese Unterschiede bestehen im Wesentlichen in der zusätzlichen Verwendung der Schieber 27 in Fig. 17, welche in Fig. 16 weggelassen sind. Mittels dieser Schieber 27 in Fig. 17 können ein oder bei entsprechender Entfernung der Schieber 27 auch mehrere Trennkörper 5 zwischen den Schiebern 27 eingesperrt werden. Vor allem kann der Bereich zwischen den Schiebern 27 aber, wie auch in den Varianten gemäß Fig. 13 und 14, dazu genutzt werden, einen oder mehrere Trennkörper 5 aus dem Behälterhohlraum 4 zu entnehmen oder in diesen einzuführen. Hierzu kann in der Behälterwand 3 in hier nicht dargestellter Art und Weise im Bereich zwischen den beiden Schiebern 27 eine Art Tür oder dergleichen oder nur ein entfernbarer und wiederbefestigbarer Wandbereich bzw. Deckel vorgesehen sein, um so die Behälterwand 3 vorübergehend öffnen zu können, um einen Trennkörper 5 herauszunehmen oder in den Behälterhohlraum 4 einzuführen. Während des Herausnehmens oder Einführens des Trennkörpers 5 sind die Schieber 27 geschlossen. Ist das Einführen und/oder Entnehmen abgeschlossen, so wird die Behälterwand 3 wieder geschlossen. Die Schieber 27 können dann wieder geöffnet werden. Das Schleusensystem gemäß Fig. 17 kann dann ansonsten wie bezüglich Fig. 16 beschrieben verwendet werden.

Für die Wahl der Positionsmesssensoren 17 und der Temperaturmesseinrichtung 18 gibt es beim Stand der Technik zahlreiche Möglichkeiten. Bei den Positionsmesssensoren 17 handelt es sich vorzugsweise um berührungslos messende Systeme, welche mittels Messung eines Magnet- oder elektrischen Feldes oder eines elektromagnetischen Feldes feststellen können, ob der Trennkörper 5 sich jeweils in der Nähe des Positionsmesssensors 17 befindet oder nicht. Für die Temperaturmesseinrichtung 18 gibt es beim Stand der Technik zahlreiche Möglichkeiten auf die hier nicht im Detail eingegangen werden muss.

Soweit dies für den jeweils im Behälter 2 durchgeführten Prozess zur Abtötung von Keimen und/oder Krankheitserregern zielführend ist, können auch hier nicht dargestellte aber an sich bekannte Druckmesssensoren eingesetzt werden, um den Druck im pumpfähigen Substrat in der Zuführleitung 19, im Behälterhohlraum 4 und/oder in der Abführleitung 20 zu überwachen. Zur Druckregelung kann zum Beispiel ein Druckregelventil in der Abführleitung 20 oder sonst irgendwo an geeigneter Stelle eingesetzt werden. Z.B. können bei einer Sterilisation Drücke bei 3 bar erreicht werden. Ein entsprechendes Druckregelventil kann z.B. in der Abführleitung 20 in Strömungsrichtung gesehen hinter dem Wärmetauscher 16 angeordnet sein.

### LEGENDE zu den Hinweisziffern:

- 1: Vorrichtung
- 2: Behälter
- 3: Behälterwand
- 4: Behälterhohlraum
- 5: Trennkörper
- 6: Teilvolumen
- 7: Teilvolumen
- 8: Membran
- 9: Rand
- 10: Befestigungsbereich
- 11: Außenkontur
- 12: Innenfläche
- 13: Dichtlippe
- 14: Borsten
- 15: Heizeinrichtung
- 16: Wärmetauscher
- 17: Positionsmesssensor
- 18: Temperaturmesseinrichtung
- 19: Zuführleitung
- 20: Abführleitung
- 21: Pumpe
- 22: Ventil
- 23: Strömungsrichtung
- 24: erste Drehventilplatte
- 25: zweite Drehventilplatte
- 26: Drehventil
- 27: Schieber
- 28: Zwischenbereich
- 29: Anschluss
- 30: Schubvorrichtung
- 31: Rückhaltesperre
- 32: zusätzliche Rückhaltesperre
- 33: Stange
- 34: Durchführung
- 35: Antrieb

## Patentansprüche

1. Vorrichtung (1) zum Abtöten von Keimen und/oder Krankheitserregern in einem pumpfähigen Substrat, wobei die Vorrichtung (1) zumindest einen Behälter (2) mit einem, von einer Behälterwand (3) des Behälters (2) umgebenen, Behälterhohlraum (4) zur Aufnahme des pumpfähigen Substrats aufweist, und im Behälterhohlraum (4) zumindest ein innerhalb des Behälterhohlraums (4) verlagerbarer Trennkörper (5) der Vorrichtung (1) angeordnet ist, wobei der Trennkörper (5) den Behälterhohlraum (4) in zwei voneinander getrennte Teilvolumina (6, 7) zur Aufnahme des pumpfähigen Substrats trennt, **dadurch gekennzeichnet, dass** die Vorrichtung (1) ein Positionsmesssystem zur Bestimmung der Position des Trennkörpers (5) im Behälterhohlraum (4) in Abhängigkeit von der Zeit aufweist.

2. Vorrichtung (1) nach Anspruch 1, **dadurch gekennzeichnet, dass** der Trennkörper (5) eine Barriere zur Verhinderung der Vermischung von pumpfähigem Substrat aus einem der Teilvolumina (6) mit pumpfähigem Substrat aus dem anderen der Teilvolumina (7) ist.

3. Vorrichtung (1) nach Anspruch 1 oder 2, **dadurch gekennzeichnet, dass** der Trennkörper (5) gegen die Behälterwand (3) abgedichtet ist.

4. Vorrichtung (1) nach einem der Ansprüche 1 bis 3, **dadurch gekennzeichnet, dass** der Trennkörper (5) innerhalb des Behälterhohlraums (4) verschiebbar angeordnet ist.

5. Vorrichtung (1) nach einem der Ansprüche 1 bis 3, **dadurch gekennzeichnet, dass** der Trennkörper (5) eine flexibel dehnbare Membran (8) ist oder zumindest aufweist.

6. Vorrichtung (1) nach Anspruch 5, **dadurch gekennzeichnet, dass** die Membran (8) in zumindest einem Befestigungsbereich (10) der Membran (8) ortsfest an der Behälterwand (3) fixiert ist.

7. Vorrichtung (1) nach einem der Ansprüche 1 bis 4, **dadurch gekennzeichnet, dass** der Trennkörper (5) von der Behälterwand (3) geführt aber ansonsten von der Behälterwand (3) losgelöst ist.

8. Vorrichtung (1) nach Anspruch 7, **dadurch gekennzeichnet, dass** eine Außenkontur (11) des Trennkörpers (5) umfangsgeschlossen an einer den Behälterhohlraum (4) umgebenden Innenfläche (12) der Behälterwand (3) anliegt.

9. Vorrichtung (1) nach Anspruch 7 oder 8, **dadurch gekennzeichnet, dass** der Trennkörper (5) an seiner Außenkontur (11) zumindest eine Dichtlippe (13) und/oder zumindest eine Anordnung von Borsten (14) zur Anlage an einer den Behälterhohlraum (4) umgebenden Innenfläche (12) der Behälterwand (3) aufweist.

10. Vorrichtung (1) nach einem der Ansprüche 1 bis 9, **dadurch gekennzeichnet, dass** der Behälterhohlraum (4) längserstreckt ausgebildet ist.

11. Vorrichtung nach Anspruch 10, **dadurch gekennzeichnet, dass** der Behälterhohlraum (4) zumindest bereichsweise oder vollständig rohrförmig ausgebildet ist.

12. Vorrichtung (1) nach einem der Ansprüche 1 bis 11, **dadurch gekennzeichnet, dass** die Vorrichtung (1) eine Heizeinrichtung (15) zum Heizen des pumpfähigen Substrats aufweist.

13. Vorrichtung (1) nach einem der Ansprüche 1 bis 12, **dadurch gekennzeichnet, dass** die Vorrichtung (1) eine wärmende und/oder thermisch dämmende Ummantelung für den Behälter (2) aufweist.

14. Vorrichtung (1) nach einem der Ansprüche 1 bis 13, **dadurch gekennzeichnet, dass** die Vorrichtung (1) einen Wärmetauscher (16) zur Wärmeübertragung vom aus dem Behälter (2) abströmenden pumpfähigen Substrat auf das dem Behälter (2) zuströmende pumpfähige Substrat aufweist.

15. Vorrichtung (1) nach einem der Ansprüche 1 bis 14, **dadurch gekennzeichnet, dass** die Vorrichtung (1) eine Temperaturmesseinrichtung zur Bestimmung der Temperatur des aus dem Behälter (2) abströmenden pumpfähigen Substrats und/oder auf den Behälter (2) zuströmenden pumpfähigen Substrats aufweist.

## Claims

1. Device (1) for killing germs and/or pathogens in a pumpable substrate, the device (1) comprising at least one container (2) having a container cavity (4), which is enclosed by a container wall (3) of the container (2), for holding the pumpable substrate, and at least one separating member (5) of the device (1) being arranged in the container cavity (4), said separating member being movable within the container cavity (4), the separating member (5) separating the container cavity (4) into two separate part-volumes (6, 7) for holding the pumpable substrate, **characterised in that** the device (1) comprises a position measurement system for determining the position of the separating member (5) in the container cavity (4) as a function of time.

2. Device (1) according to claim 1, **characterised in that** the separating member (5) is a barrier for preventing pumpable substrate from one of the part-volumes (6) mixing with pumpable substrate from the other part-volume (7).

3. Device (1) according to either claim 1 or claim 2, **characterised in that** the separating member (5) is sealed against the container wall (3).

4. Device (1) according to any of claims 1 to 3, **characterised in that** the separating member (5) is arranged displaceably within the container cavity (4).

5. Device (1) according to any of claims 1 to 3, **characterised in that** the separating member (5) is or at least comprises a flexibly extensible membrane (8).

6. Device (1) according to claim 5, **characterised in that** the membrane (8) is secured to the container wall (3) in a stationary manner in at least one fastening region (10) of the membrane (8).

7. Device (1) according to any of claims 1 to 4, **characterised in that** the separating member (5) is guided by the container wall (3) but is otherwise detached from the container wall (3).

8. Device (1) according to claim 7, **characterised in that** an outer contour (11) of the separating member (5) abuts an inner surface (12), which encloses the container cavity (4), of the container wall (3) in a circumferentially closed manner.

9. Device (1) according to either claim 7 or claim 8, **characterised in that**, on its outer contour (11), the separating member (5) comprises at least one sealing lip (13) and/or at least one arrangement of bristles (14) for abutting an inner surface (12), which encloses the container cavity (4), of the container wall (3).

10. Device (1) according to any of claims 1 to 9, **characterised in that** the container cavity (4) is elongate.

11. Device according to claim 10, **characterised in that** the container cavity (4) is tubular at least in some regions or in its entirety.

12. Device (1) according to any of claims 1 to 11, **characterised in that** the device (1) comprises a heating apparatus (15) for heating the pumpable substrate.

13. Device (1) according to any of claims 1 to 12, **characterised in that** the device (1) comprises a heating and/or a thermally insulating sheath for the container (2).

14. Device (1) according to any of claims 1 to 13, **characterised in that** the device (1) comprises a heat exchanger (16) for transferring heat from the pumpable substrate flowing out of the container (2) to the pumpable substrate flowing into the container (2).

15. Device (1) according to any of claims 1 to 14, **characterised in that** the device (1) comprises a temperature measurement apparatus for determining the temperature of the pumpable substrate flowing out of the container (2) and/or of the pumpable substrate flowing into the container (2).

## Revendications

1. Dispositif (1) pour tuer des germes et/ou des agents pathogènes dans un substrat pouvant être pompé, le dispositif (1) étant muni d'au moins un récipient (2) avec une cavité de récipient (4) entourée par une paroi de récipient (3) du récipient (2) pour recevoir le substrat pouvant être pompé, et au moins un corps de séparation (5) du dispositif (1) étant disposé dans la cavité de récipient (4) en étant mobile à l'intérieur de ladite cavité de récipient (4), le corps de séparation (5) séparant la cavité de récipient (4) en deux volumes partiels (6, 7) séparés pour recevoir le substrat pouvant être pompé, **caractérisé en ce que** le dispositif (1) est muni d'un système de mesure de position pour déterminer la position du corps de séparation (5) dans la cavité de récipient (4) en fonction du temps.

2. Dispositif (1) selon la revendication 1, **caractérisé en ce que** le corps de séparation (5) est une barrière pour empêcher le mélange du substrat pouvant être pompé provenant de l'un des volumes partiels (6) avec le substrat pouvant être pompé provenant de l'autre des volumes partiels (7).

3. Dispositif (1) selon la revendication 1 ou 2, **caractérisé en ce que** le corps de séparation (5) est rendu étanche par rapport à la paroi de récipient (3).

4. Dispositif (1) selon l'une des revendications 1 à 3, **caractérisé en ce que** le corps de séparation (5) est disposé de manière à pouvoir coulisser à l'intérieur de la cavité de récipient (4).

5. Dispositif (1) selon l'une des revendications 1 à 3, **caractérisé en ce que** le corps de séparation (5) est une membrane (8) extensible de manière flexible, ou au moins est muni d'une telle membrane.

6. Dispositif (1) selon la revendication 5, **caractérisé en ce que** la membrane (8) est fixée de manière fixe à la paroi de récipient (3) dans au moins une zone de fixation (10) de la membrane (8).

7. Dispositif (1) selon l'une des revendications 1 à 4, **caractérisé en ce que** le corps de séparation (5) est guidé par la paroi de récipient (3) tout en étant par ailleurs détaché de la paroi de récipient (3).

8. Dispositif (1) selon la revendication 7, **caractérisé en ce qu'**un contour extérieur (11) du corps de séparation (5) est en appui périphérique avec une surface intérieure (12) de la paroi de récipient (3) entourant la cavité de récipient (4).

9. Dispositif (1) selon la revendication 7 ou 8, **caractérisé en ce que** le corps de séparation (5) est muni sur son contour extérieur (11) d'au moins une lèvre d'étanchéité (13) et/ou d'au moins un agencement de poils (14) pour l'appui contre une surface intérieure (12) de la paroi de récipient (3) entourant la cavité de récipient (4).

10. Dispositif (1) selon l'une des revendications 1 à 9, **caractérisé en ce que** la cavité de récipient (4) est allongée.

11. Dispositif selon la revendication 10, **caractérisé en ce que** la cavité de récipient (4) est au moins en partie, ou entièrement, tubulaire.

12. Dispositif (1) selon l'une des revendications 1 à 11, **caractérisé en ce que** le dispositif (1) est muni d'un dispositif de chauffage (15) pour chauffer le substrat pouvant être pompé.

13. Dispositif (1) selon l'une des revendications 1 à 12, **caractérisé en ce que** le dispositif (1) est muni d'une enveloppe chauffante et/ou thermiquement isolante pour le réservoir (2).

14. Dispositif (1) selon l'une des revendications 1 à 13, **caractérisé en ce que** le dispositif (1) est muni d'un échangeur de chaleur (16) pour le transfert de chaleur du substrat pouvant être pompé sortant du récipient (2) vers le substrat pouvant être pompé entrant dans le récipient (2).

15. Dispositif (1) selon l'une des revendications 1 à 14, **caractérisé en ce que** le dispositif (1) est muni d'un dispositif de mesure de la température pour déterminer la température du substrat pouvant être pompé sortant du récipient (2) et/ou du substrat pouvant être pompé entrant dans le récipient (2).
